Europäisches Patentamt

European Patent Office

Office européen des brevets

(11) Numéro de publication : **0 396 436 B1**

# FASCICULE DE BREVET EUROPEEN

(45) Date de publication du fascicule du brevet :
**03.08.94 Bulletin 94/31**

(51) Int. Cl.⁵ : **C12N 1/18,** C12P 21/02, C12N 15/15, C12N 15/57

(21) Numéro de dépôt : **90400838.0**

(22) Date de dépôt : **28.03.90**

(54) Souches de levure pour la production de protéines hétérologues matures, en particulier d'hirudine.

(30) Priorité : **31.03.89 FR 8904305**

(43) Date de publication de la demande :
**07.11.90 Bulletin 90/45**

(45) Mention de la délivrance du brevet :
**03.08.94 Bulletin 94/31**

(84) Etats contractants désignés :
**AT BE CH DE DK ES FR GB GR IT LI LU NL SE**

(56) Documents cités :
**EP-A- 0 252 854**
**EP-A- 0 319 944**
**EP-A- 0 327 377**
**BIOTECHNOLOGY, vol. 6, janvier 1988, pages 72-77, New York, US; G. LOISON et al.: "Expression and secretion in S. Cerevisiae of biologically active leech hirudin"**
**JOURNAL OF CELLULAR BIOCHEMISTRY, supplément 12B, janvier 1988, page 271, Alan R.Liss, Inc., New York, US; R.S. FULLER et al.: "The yeast pro-hormone cleaving enzyme, KEX2"**

(56) Documents cités :
**CELL, vol. 50, 14 août 1987, pages 573-584, Cell Press, Cambridge, MA, US; A.DMO-COWSKA et al.: "Yeast KEX1 gene encodes a putative protease with a carboxypeptidase B-like function involved in killer toxin and alpha-factor precursor processing"**

(73) Titulaire : **TRANSGENE S.A.**
**11, rue de Molsheim**
**F-67000 Strasbourg (FR)**

(72) Inventeur : **Lemoine, Yves**
**4, rue des Alisiers**
**F-67100 Strasbourg (FR)**
Inventeur : **Nguyen, Martine**
**21, rue du Paradis**
**F-67670 Wittersheim (FR)**
Inventeur : **Achstetter, Tilman**
**Uhlandweg 11**
**D-7602 Oberkirch (DE)**

(74) Mandataire : **Warcoin, Jacques et al**
**Cabinet Régimbeau**
**26, avenue Kléber**
**F-75116 Paris (FR)**

## Description

La présente invention concerne un perfectionnement apporté à la préparation de protéines hétérologues par des levures recombinantes, telles que Saccharomyces cerevisiae et plus particulièrement à la préparation d'hirudine. Elle concerne en premier lieu une nouvelle souche de levure productrice de la protéine hétérologue transformée par un vecteur permettant l'expression de la protéine sous forme mature.

Les levures sont des organismes eucaryotes unicellulaires ; le genre de levure Saccharomyces comprend des souches dont la biochimie et la génétique sont étudiées intensivement en laboratoire ; il comprend également des souches utilisées dans l'industrie alimentaire (pain, boissons alcoolisées...) et produites par conséquent en très grande quantité. La facilité avec laquelle on peut manipuler la génétique des cellules de Saccharomyces cerevisiae et la longue histoire industrielle de cette espèce en font donc un hôte de choix pour la production de protéines étrangères en utilisant les techniques de l'ADN recombinant.

Lorsque l'on cherche à obtenir des protéines d'intérêt industriel, destinées à être produites en grande quantité, il est souhaitable, afin qu'elle conserve les propriétés désirées, que la protéine soit produite essentiellement sous forme mature, c'est-à-dire dépourvue de tout acide aminé ou séquence peptidique supplémentaire demeurés fusionnés à la protéine. Ceci est particulièrement souhaitable dans le cas de l'hirudine. En effet, l'hirudine dont la source principale se trouve dans les glandes salivaires des sangsues médicinales sous forme d'un mélange de peptides de 65 à 66 acides aminés, est un inhibiteur très spécifique et très efficace de la thrombine. Il s'agit donc d'un agent thérapeutique très intéressant dont l'utilisation en clinique exige une très grande pureté d'un produit actif.

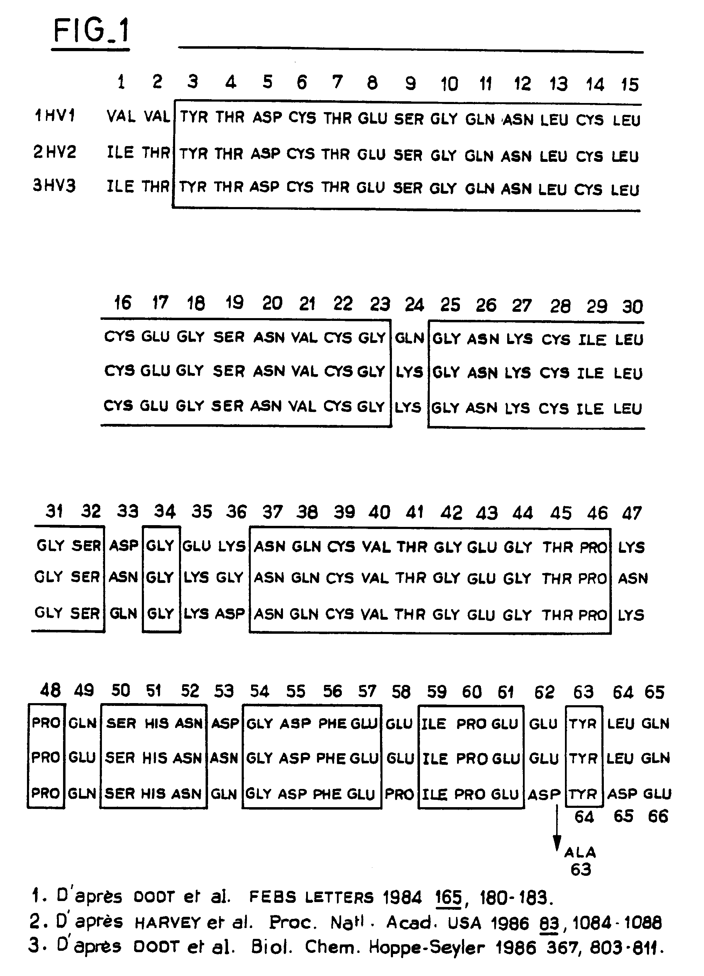

Un certain nombre de variants naturels de l'hirudine ont été identifiés et désignés par HV1, HV2, HV3. Leur structure est indiquée à la figure 1. Par la suite ces variants naturels ainsi que d'autres analogues ont été préparés par génie génétique, en particulier par fermentation de souches de S. cerevisiae comme cela est décrit par exemple dans les publications européennes de brevet EP-A-0252854 EP-A-0273800 au nom de la Demanderesse.

Comme cela a déjà été indiqué, la levure S. cerevisiae est donc particulièrement intéressante pour la production de protéines hétérologues, c'est-à-dire de protéines qui ne sont ni produites naturellement par la levure, ni nécessaires à sa croissance. En effet, cette levure est elle-même capable de sécréter quelques protéines dans le milieu de culture, correctement processées c'est-à-dire sous une forme mature. On trouve par exemple, dans le milieu de culture d'une souche de Saccharomyces cerevisiae de type sexuel alpha la phéromone sexuelle alpha.

La phéromone sexuelle alpha de levure est un peptide de 13 amino acides. Kurjan et Herskowitz (1982, Cell. 30, 933-934) ont cloné le gène structural du précurseur de la phéromone alpha (MFalphal) et ont déduit de la séquence de ce gène que ce facteur alpha de 13 amino-acides était synthétisé sous forme d'une pré-pro-protéine précurseur de 165 amino-acides. Le précurseur contient une séquence signal hydrophobe amino-terminale de 19 résidus suivis d'une séquence "pro" de 64 acides aminés contenant 3 sites de glycosylation elle même suivie de la séquence Lys-Arg précédant 4 copies du facteur alpha séparés par des peptides espaceurs.

Il a été démontré que la séquence signal est effectivement clivée au niveau du réticulum endoplasmique par une peptidase spécifique. La séquence "pro" subit une N-glycosylation initiée dans le réticulum endoplasmique. La première étape de clivage protéolytique est effectuée par le produit du gène KEX2, c'est-à-dire l'endo-protéase yscF, au niveau des signaux spécifiques Lys-Arg ou Arg-Arg. Cette maturation a probablement lieu dans l'appareil de Golgi. Il a été clairement établi que des souches de S. cerevisiae ayant subi des mutations dans le gène KEX2 ne parvenaient plus à sécréter une forme active de la protéine killer (Leibowitz and Wickner (1976) PNAS USA 2061-2065) ou de la phéromone alpha (Julius et al. (1984) Cell 37, 1075-1089) du fait d'une maturation défectueuse de ces protéines.

L'invention propose, à partir de ces travaux de nature fondamentale, d'amplifier un gène KEX2 fonctionnel dans une souche de levure productrice d'une protéine hétérologue afin d'obtenir une protéine mature.

L'invention propose en particulier des moyens pour augmenter la quantité d'hirudine mature sécrétée par une souche de levure telle que Saccharomyces cerevisiae en favorisant le processus de maturation.

L'invention a ainsi pour objet un plasmide multicopie replicable dans une levure comprenant au moins le bloc d'expression d'une protéine hétérologue suivant :
- une séquence d'ADN (Str) comportant les signaux assurant la transcription par la levure de la séquence codant pour un précurseur de la protéine maturable
- une séquence d'ADN (Spr) codant pour un peptide signal pre et/ou un peptide pro;
- une séquence d'ADN (Scl) codant pour un peptide comprenant un site de clivage protéolytique par le produit de tout ou partie du gène KEX2,
- une séquence d'ADN codant pour la protéine hétérologue, qui avec la séquence Spr forme le précurseur,

caractérisé en ce que le plasmide comporte tout ou partie du gène KEX2 menant à une augmentation de l'activité protéolytique de l'endoprotéase yscF.

Comme indiqué précédemment, l'invention s'applique tout particulièrement bien à la production d'hirudine, notamment du variant rHV2Lys47.

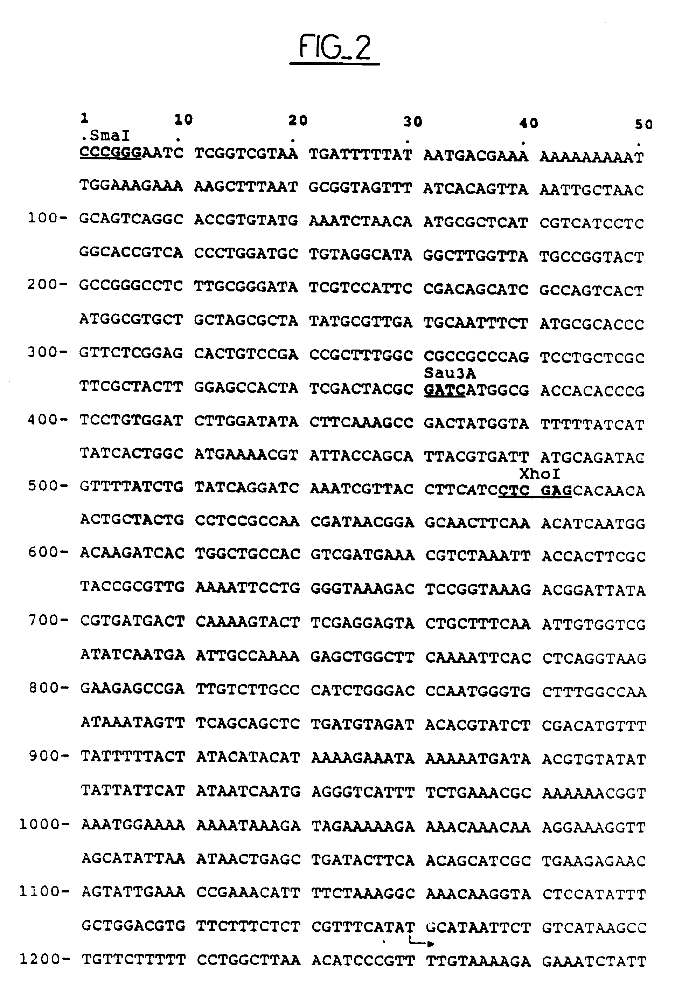

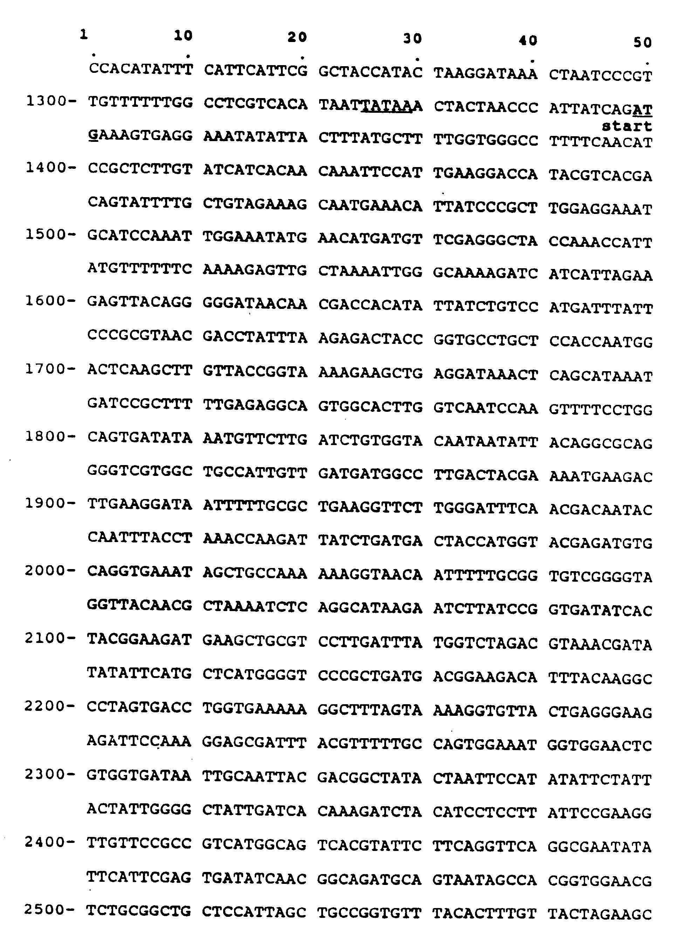

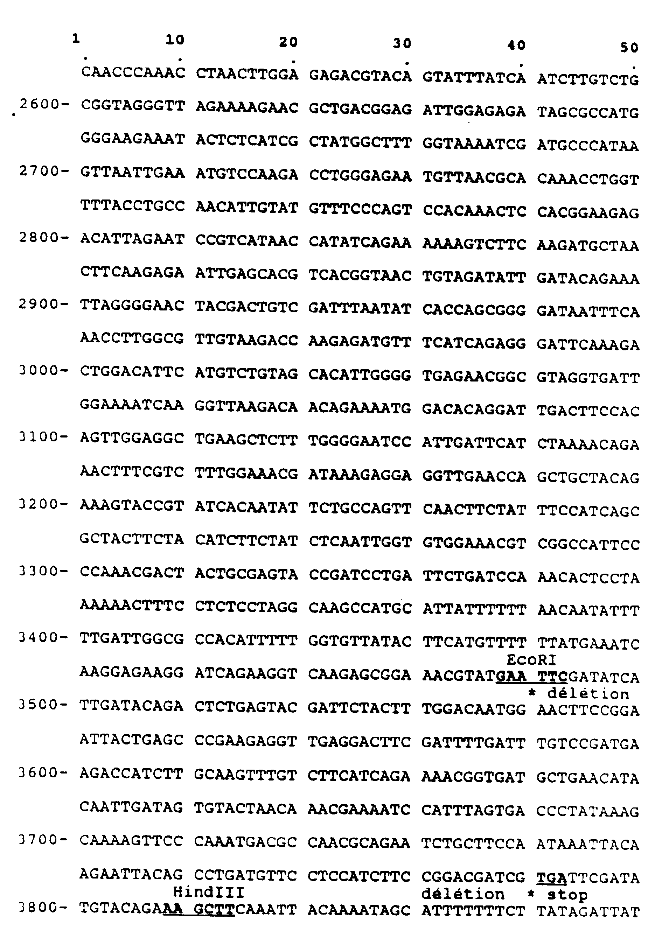

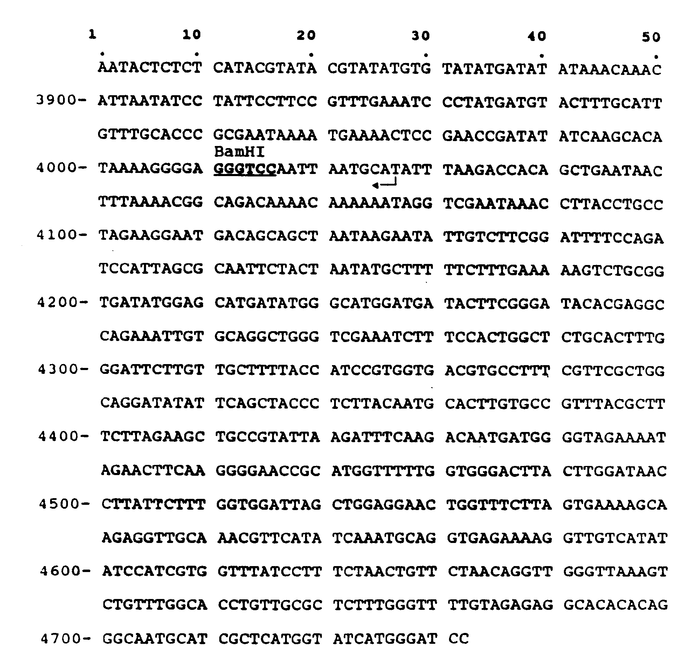

Le gène KEX2 peut être obtenu, comme cela sera décrit ci-après, par clonage à partir d'une banque d'ADN totale de levure. La séquence du gène est indiquée à la figure 2. Elle n'a pas été reprise dans la description pour ne pas l'alourdir mais il doit être entendu que cette figure 2 fait intégralement partie de la description.

Différents fragments de ce gène peuvent être utilisés selon l'invention : soit la totalité de la séquence indiquée à la figure 2 à partir de la base 381 qui correspond à la totalité de l'ADN génomique du gène KEX2; soit uniquement la séquence fonctionnelle de ce gène, c'est-à-dire celle comprise entre les bases numérotées 1349 et 3793 (délimitée par les codons ATG et TGA d'initiation et de terminaison de traduction) sur la figure 2; soit même une partie seulement de la séquence codante, par exemple la partie comprise entre les bases numérotées 1349 et 3488 sur la figure 2.

La présence du fragment situé entre les bases numérotées 3790 et 4010 de la figure 2 à l'extrémité 3' du segment du gène KEX 2 améliore l'expression du gène en jouant un rôle de terminateur et de stabilisateur de la construction.

Il est souhaitable de déleter le fragment compris entre les bases numérotées 3488 et 3790 sur la figure 2, qui aurait un rôle destabilisant au niveau de l'ARN messager.

L'amplification de l'expression du gène peut être réalisée de plusieurs façons. Dans une première variante, une ou plusieurs copies de tout ou partie du gène KEX2 sont intégrées directement dans le gènome de la levure. Dans une seconde variante, une ou plusieurs copies de tout ou partie du gène KEX2 sont insérées dans le vecteur d'expression de la protéine hétérologue, en dehors du bloc d'expression proprement dit. Dans la suite de la description on s'intéressera plus particulièrement à la seconde variante.

Le produit du gène KEX2 effectue les clivages protéolytiques de façon spécifique. C'est pourquoi les séquences Scl doivent être choisies parmi un nombre limité de séquences possibles. On choisira préférentiellement les peptides Lys-Arg ou Arg-Arg ou même Ser-Leu-Asp. Ces séquences Scl peuvent être ou non précédées d'une séquence "pro" c'est-à-dire qu'elles peuvent être on non associées à un système de sécrétion complexe. En effet, dès lors que le site de clivage protéolytique par le produit du gène KEX2 est présent on peut s'attendre à ce que la maturation de la protéine ait lieu, quel que soit le système de sécrétion utilisé : une séquence codant pour un peptide signal pré ou une telle séquence associée à une séquence pro (séquence pré-pro).

Une construction particulièrement intéressante pour la production d'hirudine est celle qui associe la séquence pré-pro de la phéromone alpha de levure au site de clivage Lys-Arg ou éventuellement Arg-Arg. Enfin, il est également intéressant d'utiliser comme séquence Str un promoteur fort dans la levure, de façon à transcrire une quantité importante d'ARNm correspondant au précurseur de la protéine maturable et à utiliser le gène KEX2 avec un maximum d'efficacité. Comme promoteur fort, on peut citer par exemple le promoteur de la phéromone alpha de levure ou encore le promoteur PGK de levure.

Enfin, les vecteurs d'expression pourront présenter, après la séquence d'ADN codant pour la protéine mature, une séquence de terminateur de levure, par exemple celui du gène PGK.

Les vecteurs d'expression seront en général des plasmides à replication autonome. Ces plasmides comporteront des éléments assurant leur replication, c'est-à-dire une origine de réplication telle que celle du plasmide 2μ de levure. En outre, le plasmide pourra comporter des éléments de sélection tels que le gène URA3 ou LEU2 qui assurent la complémentation des mutants de levure ura3 ou leu2. En particulier on pourra avantageusement utiliser le gène URA3 délété de son promoteur.

Ces plasmides peuvent également comporter des éléments assurant leur réplication dans E. coli, lorsque le plasmide doit être un plasmide navette, par exemple une origine de réplication telle que celle du pBR322, un gène marqueur tel que Ap$^R$ et/ou d'autres éléments connus de l'homme du métier.

Parmi toutes les souches de levure envisageables on utilisera de préférence les souches du genre Saccharomyces notamment de l'espèce cerevisiae. Lorsque le promoteur est celui du gène MFalphal, la levure sera de préférence de type sexuel MATalpha. on utilisera, par exemple, une souche de génotype ura3 ou leu2 ou autre, complémentée par le plasmide pour assurer le maintien du plasmide dans la levure par une pression de sélection appropriée.

Enfin, l'invention a pour objet un procédé de préparation d'une protéine hétérologue mature, par des levures selon lequel on met en culture dans un milieu approprié les souches selon l'invention et on récupère la protéine mature sécrétée dans le milieu de culture.

Plus particulièrement, l'invention concerne un procédé de sécrétion de l'hirudine notamment le variant rHV2Lys47, sous forme mature à partir des souches de levures selon l'invention.

Les exemples ci-après permettront de mettre en évidence d'autres caractéristiques et avantages de la présente invention. Ces exemples seront illustrés par les figures suivantes :
- la figure 1 représente la séquence des variants HV1, HV2, HV3 de l'hirudine.
- la figure 2 représente la séquence de l'ADN génomique du gène KEX2 de la levure,
- la figure 3 représente schématiquement le plasmide pTG2958.
- la figure 4 représente schématiquement la structure des vecteurs M13TG3839 et M13TG3841.
- la figure 5 représente schématiquement la carte de restriction des fragments du gène KEX2 utilisés.

## EXEMPLE 1 : Clonage du gène KEX2

Ce clonage est réalisé par complémentation phénotypique d'une souche mutée kex2-1. La mutation du gène KEX2 se manifeste dans une souche de levure MATalpha par la l'absence de sécrétion de phéromone alpha active et dans une souche de levure possédant l'ARN killer (K+) par l'absence de sécrétion de la toxine killer. La souche TGY38.1 est utilisée comme souche réceptrice pour le clonage du gène KEX2. Pour obtenir cette souche, les souches 80 (MATa, kex2-1, adel, ura1, [KIL-k] K- R+) [Yeast Genetic Stock Center, Berkeley CA 94720] et TGY1sp4 (MATalpha, his3, ura3) sont tout d'abord croisées. Le diploïde obtenu TGY33 est mis à sporuler. Une spore TGY33.1 est ensuite caractérisée, son génotype est MATa, his3, ura3, kex2-1. Un croisement en retour est effectué avec TGY1sp4. Le diploïde obtenu TGY38 est mis à sporuler. Après germination, un ségrégeant TGY38.1 (MATa, ura3, his3, kex2-1) ayant une meilleure efficacité de transformation est isolé.

Un clone génomique, pTG2809 est obtenu à partir de la banque génomique de levure (fragments d'ADN chromosomique partiellement digéré par Sau3A insérés dans le site BamHI de pFL1 [Parent, S.A. et al. Yeast 1 (1985)]. Ce clone (un (K+) parmi dix mille transformants URA+ de la souche TGY38.1) est isolé sur la base de sa capacité à supprimer le défaut fonctionnel dû à la mutation kex2-1 : sécrétion d'une protéine killer active.

En analysant la séquence d'ADN (figure 2) on confirme que la séquence codante de 2442 paires de base est contenu dans l'insert (délimitée par les codons d'initiation et de terminaison de traduction sur la figure 2). La séquence correspond à la séquence publiée du gène KEX2 [Mizuno K. et al. Biochem. Biophys. Res. Comm. 156 (1988)]. L'analyse de la séquence codante du gène KEX2 révèle une information correspondant à un polypeptide de 814 acides aminés. La protéine contiendrait une séquence signal putative d'environ 21 acides aminés. Une partie hydrophobe de 21 acides aminés localisée près de la partie C-terminale peut être mise en évidence (position 679-699). Le fragment EcoRI qui complémente la déficience due à la mutation kex2-1, code pour une forme tronquée en C-terminal du produit du gène KEX2 représentant la partie comprise entre les bases numérotées 1349 et 3488 (figure 2).

## EXEMPLE 2 : Construction des plasmides pTG3848, pTG3855, pTG3887, pTG3890 et pTG3872

### A. Construction de M13TG3841

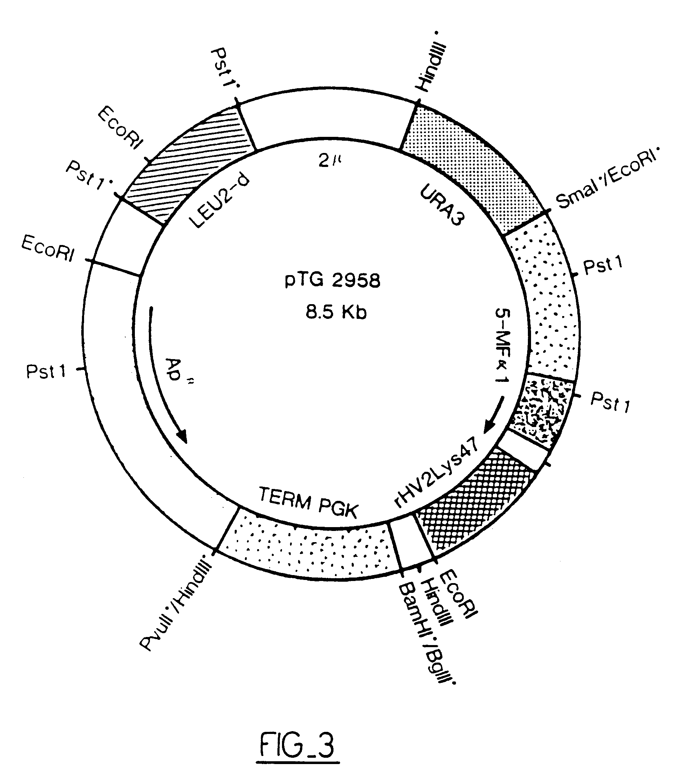

Le plasmide pTG2958 (figure 3) est peu différent du plasmide pTG1833 décrit dans la publication européenne de brevet EP-A-252854 porteur de la séquence codante pour rHV2Asp47. Le plasmide pTG2958 ne contient pas le site de restriction <u>HindIII</u> artificiellement introduit. Le plasmide pTG2958 contient :
- un fragment de 547 paires de bases correspondant à la région 5' du gène MFalphal (contenant le promoteur, la séquence codant pour le peptide signal, la région "pro" et une séquence codant por le peptide Lys-Arg),
- un fragment de 234 paires de bases contenant l'ADN complémentaire de rHV2Lys47,
- un fragment de 243 paires de bases comprenant le terminateur PGK de levure,
- le fragment PvuII-EcoRI de pBR322 comprenant entre autres l'origine de réplication de ce plasmide et le gène de résistance à l'ampicilline (2292 paires de bases),
- le fragment EcoRI-HindIII du plasmide 2μ de la levure (forme B), contenant le gène LEU2 de levure, sous forme délétée et inséré dans le site PstI,
- un fragment HindIII-SmaI du gène URA3 de levure.

Le fragment NcoI-NcoI du vecteur pTG2958 qui porte les séquences LEU2-d, 2μ et URA3 est remplacé par le fragment NcoI-NcoI de pTG2800 décrit dans la publication européenne de brevet EP-A-O268501 qui porte les séquences du plasmide 2μ et du gène URA3 délété de son promoteur (URA3-d) pour donner pTG2877.

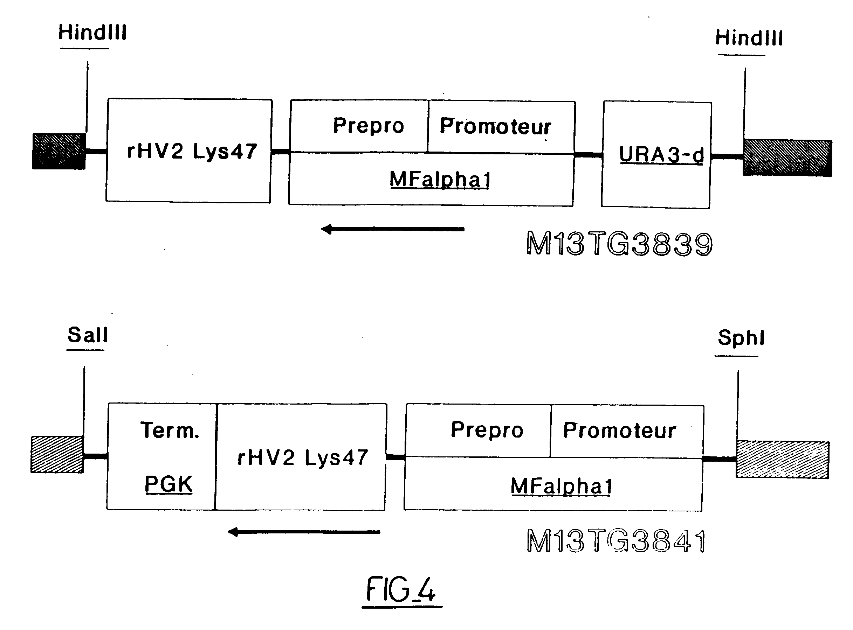

Le vecteur M13TG3839 (figure 4) dérive de M13TG103 [Kieny, M.P. et al. Gene 26 (1983)] dans lequel le fragment HindIII-HindIII de pTG2877 est introduit dans le même site. Un site de restriction SalI est introduit dans ce vecteur en aval du codon de terminaison de traduction de la région codant pour rHV2Lys47 par mutagénèse dirigée à l'aide de l'oligonucléotide suivant :

5' CAATGAAAAATGGTCGACTATCAATCATAG

pour donner M13TG3839 SalI. Un site de restriction SphI est alors introduit en amont de la cassette d'expression en éliminant la séquence URA3-d par mutagénèse dirigée à l'aide de l'oligonucléotide suivant :

5' GACGGCCAGTGAATTGGCATGCTATTGATAAGATTTAAAG

pour donner M13TG3840.

Le vecteur M13TG131 [Kieny M.P. et al. Gene 26 (1983)] est clivé par PstI, les extrémités rendues franches par traitement à l'aide du fragment Klenow de l'ADN polymérase I pour être ensuite religué sur lui même pour donner M13TG3160. Ce vecteur est ensuite clivé par SmaI et EcoRV puis religué pour donner M13TG3149.

Le fragment SphI-SalI de M13TG3840 (décrit ci-dessus) portant la cassette d'expression de rHV2Lys47 (sans séquence terminatrice de transcription) est introduit dans le site SphI-SalI de M13TG3149 pour donner M13TG3841 (figure 4).

B. Construction du plasmide pTG3848

Le plasmide pTG848 décrit dans la publication européenne de brevet EP-A-0252854 est digéré par BglII puis religué pour donner pTG2886. Le grand fragment HindIII-EcoRI de pTG2886 est ligué en présence de ligase T4 au fragment HindIII-EcoRI de 2,1 kb du plasmide pFL1 [Parent, S.A. et al. Yeast 1 1985] qui porte la séquence du plasmide 2μ de S. cerevisiae pour donner le plasmide pTG2886 LEU2-d, URA3-d. Le fragment HindIII de 0,9 kb du plasmide pTG2800 décrit dans la publication européenne de brevet EP-A-0258501 portant le gène URA3-d est alors inséré dans le site HindIII de ce plasmide pour donner pTG2886 URA3-d, delta LEU2-d. Le fragment SmaI-BglII de M13TG131 [Kieny et al. Gene 26; 1983] qui possède plusieurs sites de restriction est ensuite introduit dans ce plasmide pour donner pTG3828.

Le plasmide pTG3828 est digéré par les enzymes de restriction SphI-SalI puis le fragment SphI-SalI du vecteur M13TG3841 (figure 4 ) est inséré dans ce site pour donner le plasmide pTG3848. Ce plasmide comporte :
   - la séquence du gène URA3 délétée de son promoteur,
   - le promoteur du gène MFalphal,
   - la séquence pré-pro de MFalphal,
   - la séquence codant pour rHV2Lys47
   - le terminateur de transcription du gène PGK de levure
   - un fragment de pBR322 qui permet la réplication et la sélection chez Escherichia coli,
   - un fragment du plasmide 2μ qui possède les éléments structuraux nécessaires à la réplication et à l'équipartition mitotique dans la levure.

C. Construction de pTG3855, pTG3887, pTG3890 et pTG3872

Ces plasmides dérivent de pTG3848 ouvert au site EcoRI. Un site artificiel BamHI est créé en aval du codon stop du gène KEX2 par mutagénèse dirigée à l'aide de l'oligonucléotide suivant :

5' TAAAAGGGGAGGATCCAATTAATGC 3'

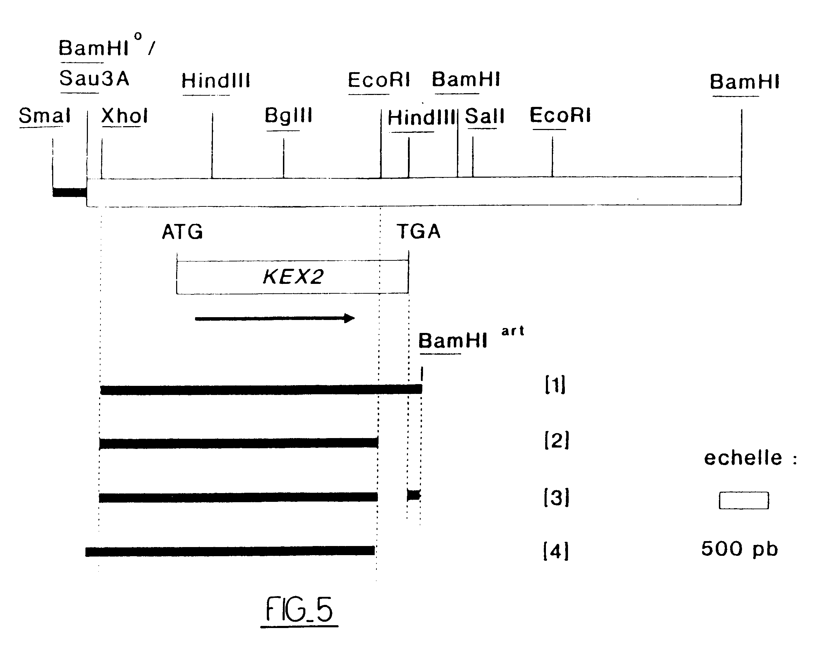

Le fragment XhoI-BamHI (coordonnées 538 à 4011, figure 2) de pTG2809 comprenant tous les éléments structuraux du gène KEX2 (figure 5[1]) dont les extrémités sont rendues franches par l'ADN polymérase de Klenow est inséré dans le site EcoRI de pTG3848 ayant subi le même traitement pour donner le plasmide pTG3855. Le fragment XhoI-EcoRI (coordonnées 538 à 3488, figure 2) de pTG2809 (figure 5[2]) dont les extrémités sont rendues franches par l'ADN polymérase de Klenow est inséré dans le site EcoRI de pTG3848 ayant subi le même traitement pour donner le plasmide pTG3887 et le plasmide pTG3883 suivant l'orientation du fragment. Dans le cas du plasmide pTG3887 la transcription de KEX 2 se fait vers la séquence bactérienne (pBR322) portée par le plasmide alors que pour le plasmide pTG3883 la transcription se fait vers la séquence 2μ du plasmide.

Afin de déléter la partie du gène KEX 2 comprise entre le site EcoRI et le TGA, une mutagénèse dirigée est réalisée en utilisant l'oligonucléotide de séquence :

5' AAGAGCGGAAACGTATGAATGATTCGATATGTACAGAAAG 3'

Le fragment XhoI-BamHI artificiel (coordonnées 538 à 4011, figure 2) de pTG2809 ainsi transformé (figure 5[3]) dont les extrémités sont rendues franches par l'ADN polymérase de Klenow est ensuite inséré dans le site EcoRI de pTG3848 ayant subi le même traitement pour donner le plasmide pTG3890. L'insert EcoRI-EcoRI de pTG2809 contenant le fragment Sau3A-EcoRI du gène KEX2 (coordonnées 381 à 3488, figure 2) (figure 5(4)) est inséré dans le site EcoRI de pTG3848 pour donner le plasmide pTG3872.

EXEMPLE 3 : Production de rHV2Lys47 en fonction du plasmide utilisé

Une souche de levure de l'espèce Saccharomyces cerevisiae de génotype MATalpha, ura3-251,373,328, leu2-3,112, his3, pep4-3 est transformée par les plasmides pTG3848, pTG3872, pTG3855, pTG3887 et pTG3890 par la méthode de l'acétate de lithium (Ito, H. et al. J. Bactériol. 153; 1983] et les prototrophes Ura+ sont sélectionnés. Ils sont ensuite mis en culture en erlenmeyer à 30°C sur un milieu sélectif (0,7% de bases azotées pour levures (Yeast Nitrogen Base), 0,5% de casamino acides et 1% de glucose). Après 48 heures de culture, on sépare cellules et surnageant par centrifugation et l'activité inhibitrice de la thrombine est déterminée dans le surnageant en utilisant le test colorimétrique (activité protéolytique sur un substrat synthétique, le chromozyme TH - Boehringer Mannheim). Le tableau 1 présente les résultats des dosages; chaque valeur correspond à la moyenne de deux expériences indépendantes. L'activité de rHV2Lys47 est exprimée en ATU/A600 des cellules (1-2x10$^7$ cellules/ml correspondent à 1 unité A600 nm).

## Tableau 1

| Plasmide | KEX2 | ATU/A600 |
|---|---|---|
| pTG3848 | - | 8 |
| pTG3872 | [4] | 23 |
| pTG3855 | [1] | 34 |
| pTG3887 | [2] | 34 |
| pTG3890 | [3] | 40 |

La présence du gène KEX2 sur les constructions selon l'invention permet une augmentation de la production de rHV2Lys47 sous forme active d'un facteur cinq.

EXEMPLE 4 : Activité spécifique de l'endo-protéase yscF en fonction du plasmide utilisé.

On récupère les extraits bruts de culture de souches de levures transformées par les plasmides pTG3848, pTG3855, pTG3883 et pTG3890 selon les procédures décrites précédemment. L'activité spécifique de l'endo-protéase yscF est déterminée par la méthode décrite dans la publication de Achstetter T. et Wolf D.H. (1985) (EMBO J. 4 ; p 173-177). Cette méthode est légèrement modifiée, en effet on n'utilise pas de Triton contrairement à ces auteurs.

Le tableau 2 présente les résultats de ces dosages. L'activité spécifique de yscF est exprimée en mU/mg de protéine. Les protéines sont dosées par une méthode colorimétrique en utilisant la trousse commercialisée par BIORAD.

TABLEAU   2

| Plasmide | KEX 2 forme | Activité spécifique |
|----------|-------------|---------------------|
| pTG3848 | – | 2,8 |
| pTG3855 | (1) | 43,7 |
| pTG3883 | (2) | 20,8 |
| pTG3890 | (3) | 74,0 |

La présence du gène KEX 2 sur les constructions selon l'invention permet d'augmenter l'activité spécifique de l'endoprotéase yscF produite. De ces résultats on peut conclure que le fragment compris entre le site EcoRI de KEX 2 et le TGA (de la base 3488 à la base 3790 ; figure 2) jouerait un rôle destabilisant au niveau de l'ARN messager. Par contre, le fragment TGA-BamHI (de la base 3790 à la base 4010 ; figure 2) jouerait le rôle de terminateur et stabiliserait ainsi la construction. La forme de l'endo-protéase yscF obtenue à l'aide du plasmide pTG3890 correspond donc à une forme dont l'activité spécifique est augmentée par rapport à la forme de référence.

**Revendications**

**Revendications pour les Etats contractants suivants : AT, BE, CH, LI, DE, DK, FR, GB, IT, LU, NL, SE**

1. Plasmide multicopie réplicable dans une levure comprenant au moins le bloc d'une protéine hétérologue suivant :
   - une séquence d'ADN (Str) comportant les signaux assurant la transcription par la levure de la séquence codant pour un précurseur de la protéine maturable,
   - une séquence d'ADN (Spr) codant pour un peptide signal pre et/ou un peptide pro,
   - une séquence d'ADN (Scl) codant pour un peptide comprenant un site de clivage protéolytique par le produit de tout ou partie du gène KEX2,
   - une séquence d'ADN codant pour la protéine hétérologue, qui avec la séquence Spr forme le précurseur,
   caractérisé en ce que le plasmide comporte tout ou partie du gène KEX2 menant à une augmentation de l'activité protéolytique de l'endoprotéase yscF.

2. Plasmide selon la revendication 1 caractérisée en ce que ladite protéine est l'hirudine.

3. Plasmide selon la revendication 2 caractérisée en ce que l'hirudine est le variant rHV2Lys47.

4. Plasmide selon l'une des revendications 1 à 3 caractérisée en ce qu'il comporte la partie du gène KEX2 dont la séquence est représentée à la figure 2.

5. Plasmide selon la revendication 4 caractérisée en ce qu'il comporte la séquence fonctionnelle du gène

7

KEX2, comprise entre les bases numérotées 1349 et 3793 sur la figure 2.

6. Plasmide selon la revendication 5 caractérisée en ce qu'il comporte la séquence fonctionnelle du gène KEX2 comprise entre les bases numérotées 1349 et 3488 sur la figure 2.

7. Plasmide selon la revendication 4, caractérisé en ce qu'il comporte la partie du gène KEX 2 comprise entre les bases numérotées 538 et 4010 sur la figure 2, et en ce qu'elle est délétée de la séquence comprise entre les bases numérotées 3488 et 3790 sur la figure 2.

8. Plasmide selon l'une des revendications 4 à 7, caractérisée en ce que le segment du gène KEX 2 comporte à l'extrémité 3′ un fragment terminateur correspondant à la séquence comprise entre les bases numérotées 3790 et 4010 sur la figure 2.

9. Plasmide selon la revendication 8, caractérisée en ce que la séquence comprise entre les bases numérotées 3790 à 4010 sur la figure 2 joue un rôle de stabilisateur.

10. Plasmide selon l'une des revendications précédentes caractérisée en ce que la séquence Scl est une séquence codant pour un peptide Lys-Arg ou Arg-Arg.

11. Plasmide selon l'une des revendication 1 à 10 caractérisée en ce que la séquence Str est un promoteur fort dans la levure.

12. Plasmide selon la revendication 11 caractérisée en ce qu'il s'agit du promoteur de la phéromone alpha de levure.

13. Plasmide selon l'une des revendications 2 à 11 caractérisée en ce que la séquence Spr est la séquence pré-pro du gène MFalphal de levure.

14. Plasmide selon la revendication 17 caractérisée en ce que la séquence Spr est suivie d'une séquence codant pour un peptide Lys-Arg ou Arg-Arg.

15. Souche de levure caractérisée en ce qu'elle comporte un plasmide selon l'une des revendications 1 à 14

16. Souche selon la revendications 15 caractérisée en ce qu'il s'agit d'une souche de <u>Saccharomyces cerevisiae.</u>

17. Procédé de préparation d'hirudine mature par des levures caractérisé en ce qu'on met en culture, dans un milieu approprié, une souche de levure selon l'une des revendications 15 à 16 et on récupère l'hirudine mature obtenue.

18. Procédé selon la revendication 17, caractérisé en ce que l'hirudine est le variant rNV2Lys47.

**Revendications pour les Etats contractants suivants : ES, GR**

1. Procédé de préparation d'un plasmide multicopie replicable dans une levure comprenant au moins le bloc d'expression d'une protéine hétérologue suivant :
   . une séquence d'ADN (Str) comportant les signaux assurant la transcription par la levure de la séquence codant pour un précurseur de la protéine maturable,
   . une séquence d'ADN (Spr) codant pour un peptide signal pre et/ou un peptide pro,
   . une séquence d'ADN (Scl) codant pour un peptide comprenant un site de clivage protéolytique par le produit de tout ou partie du gène KEX2,
   . une séquence d'ADN codant pour la protéine hétérologue, qui avec la séquence Spr forme le précurseur,
   caractérisé en ce que l'on insère dans le plasmide tout ou partie du gène KEX2 menant à une augmentation de l'activité protéolytique de l'endoprotease yscF.

2. Procédé selon la revendication 1 caractérisé en ce que ladite protéine est l'hirudine.

3. Procédé selon la revendication 2 caractérisé en ce que l'hirudine est le variant rHV2Lys47.

**4.** Procédé selon l'une des revendications 1 à 3 caractérisé en ce que la partie du gène KEX2 a la séquence qui est représentée à la figure 2.

**5.** Procédé selon la revendication 4 caractérisé en ce que la partie du gène KEX2 comporte la séquence fonctionnelle du gène KEX2, comprise entre les bases numérotées 1349 et 3793 sur la figure 2.

**6.** Procédé selon la revendication 5 caractérisé en ce que la partie du gène KEX2 comporte la séquence fonctionnelle du gène KEX2 comprise entre les bases numérotées 1349 et 3488 sur la figure 2.

**7.** Procédé selon la revendication 4 caractérisé en ce que la partie du gène KEX2 est comprise entre les bases numérotées 538 et 4010 sur la figure 2, et en ce qu'elle est délétée de la séquence comprise entre les bases numérotées 3488 et 3790 sur la figure 2.

**8.** Procédé selon l'une des revendications 4 à 7 caractérisé en ce que la partie du gène KEX2 comporte à l'extrémité 3' un fragment terminateur correspondant à la séquence comprise entre les bases numérotées 3790 et 4010 sur la figure 2.

**9.** Procédé selon la revendication 8, caractérisé en ce que la séquence comprise entre les bases numérotées 3790 et 4010 sur la figure 2 joue un rôle de stabilisateur.

**10.** Procédé selon l'une des revendications précédentes caractérisé en ce que la séquence Scl est une séquence codant pour un peptide Lys-Arg ou Arg-Arg.

**11.** Procédé selon l'une des revendications 1 à 10 caractérisé en ce que la séquence Str est un promoteur fort dans la levure.

**12.** Procédé selon la revendication 11 caractérisé en ce qu'il s'agit du promoteur de la phéromone alpha de levure.

**13.** Procédé selon l'une des revendications 2 à 11 caractérisé en ce que la séquence Spr est la séquence pre-pro du gène MFalphal de levure.

**14.** Procédé selon la revendication 17 caractérisé en ce que la séquence Spr est suivie d'une séquence codant pour un peptide Lys-Arg ou Arg-Arg.

**15.** Procédé de préparation d'une souche de levure caractérisé en ce qu'elle est transformée par un plasmide obtenu selon l'une des revendications 1 à 14.

**16.** Souche selon la revendication 15 caractérisée en ce qu'il s'agit d'une souche de <u>Saccharomyces cerevisiae.</u>

**17.** Procédé de préparation d'hirudine mature par des levures caractérisé en ce qu'on met en culture, dans un milieu approprié, une souche de levure obtenue selon l'une des revendications 15 à 16 et on récupère l'hirudine mature obtenue.

**18.** Procédé selon la revendication 13 caractériséé en ce que l'hirudine est le variant rHV2Lys47.

**Patentansprüche**

**Patentansprüche für folgende Vertragsstaaten : DE, GB, FR, IT, NL, SE, LI, CH, BE, AT, LU, DK**

**1.** Multicopy-Plasmid, das in einer Hefe replizierbar ist, das mindestens den folgenden Block zur Expression eines heterologen Proteins enthält:
   - eine DNA-Sequenz (Str), welche die Signale aufweist, welche die Transkription der für einen Vorläufer des reifungsfähigen Proteins codierenden Sequenz durch die Hefe gewährleistet,
   - eine DNA-Sequenz (Spr), die für ein Signal-Peptid pre und/oder ein Peptid pro codiert,
   - eine DNA-Sequenz (Scl), die für ein Peptid codiert, das eine Stelle zur proteolytischen Spaltung (Schneiden) durch das Produkt des gesamten Gens KEX2 oder eines Teils desselben aufweist, und
   - eine DNA-Sequenz, die für das heterologe Protein codiert, das zusammen mit der Sequenz Spr den Vorläufer bildet,

dadurch gekennzeichnet, daß das Plasmid das gesamte Gen KEX2 oder ein Teil desselben enthält, was zu einer Erhöhung der proteolytischen Aktivität der Endoprotease yscF führt.

2. Plasmid nach Anspruch 1, dadurch gekennzeichnet, daß es sich bei dem genannten Protein um Hirudin handelt.

3. Plasmid nach Anspruch 2, dadurch gekennzeichnet, daß es sich bei dem Hirudin um die Variante rHV2Lys47 handelt.

4. Plasmid nach einem der Ansprüche 1 bis 3, dadurch gekennzeichnet, daß es den Teil des Gens KEX2 enthält, dessen Sequenz in der Fig. 2 dargestellt ist.

5. Plasmid nach Anspruch 4, dadurch gekennzeichnet, daß es die funtionelle Sequenz des Gens KEX2 zwischen den Basen Nr. 1349 und 3793 in der Fig. 2 enthält.

6. Plasmid nach Anspruch 5, dadurch gekennzeichnet, daß es die funtionelle Sequenz des Gens KEX2 zwischen den Basen Nr. 1349 und 3488 in der Fig. 2 enthält.

7. Plasmid nach Anspruch 4, dadurch gekennzeichnet, daß es den Teil des Gens KEX2 zwischen den Basen Nr. 538 und 4010 in der Fig. 2 enthält und daß daraus die Sequenz zwischen den Basen Nr. 3488 und 3790 in der Fig. 2 deletiert ist.

8. Plasmid nach einem der Ansprüche 4 bis 7, dadurch gekennzeichnet, daß das Segment des Gens KEX2 an dem 3'-Ende ein Terminator-Fragment aufweist, das der Sequenz zwischen den Basen Nr. 3790 und 4010 in der Fig. 2 entspricht.

9. Plasmid nach Anspruch 8, dadurch gekennzeichnet, daß die Sequenz zwischen den Basen Nr. 3790 und 4010 in der Fig. 2 eine Stabilisatorrolle spielt.

10. Plasmid nach einem der vorhergehenden Ansprüche, dadurch gekennzeichnet, daß die Sequenz Scl eine Sequenz ist, die für ein Peptid Lys-Arg oder Arg-Arg codiert.

11. Plasmid nach einem der Ansprüche 1 bis 10, dadurch gekennzeichnet, daß die Sequenz Str ein starker Promotor in der Hefe ist.

12. Plasmid nach Anspruch 11, dadurch gekennzeichnet, daß es sich dabei um den Promotor des $\alpha$-Pheromons von Hefe handelt.

13. Plasmid nach einem der Ansprüche 2 bis 11, dadurch gekennzeichnet, daß die Sequenz Spr die Sequenz pre-pro des Gens MFalphal von Hefe ist.

14. Plasmid nach Anspruch 13, dadurch gekennzeichnet, daß auf die Sequenz Spr eine Sequenz folgt, die für ein Peptid Lys-Arg oder Arg-Arg codiert.

15. Hefestamm, dadurch gekennzeichnet, daß er ein Plasmid nach einem der Ansprüche 1 bis 14 enthält.

16. Stamm nach Anspruch 15, dadurch gekennzeichnet, daß es sich dabei um einen Stamm von Saccharomyces cerevisiae handelt.

17. Verfahren zur Herstellung von reifem Hirudin durch Hefen, dadurch gekennzeichnet, daß man in einem geeigneten Medium einen Hefestamm nach einem der Ansprüche 15 bis 16 kultiviert (züchtet) und das erhaltene reife Hirudin daraus gewinnt (abtrennt).

18. Verfahren nach Anspruch 17, dadurch gekennzeichnet, daß es sich bei dem Hirudin um die Variante rHV2Lys47 handelt.

**Patentansprüche für folgende Vertragsstaaten : ES, GR**

1. Verfahren zur Herstellung eines Multicopy-Plasmids, das in einer Hefe replizierbar ist, das mindestens den folgenden Block zur Expression eines heterologen Proteins enthält:

- eine DNA-Sequenz (Str), welche die Signale aufweist, welche die Transkription der für einen Vorläufer des reifungsfähigen Proteins codierenden Sequenz durch die Hefe gewährleistet,
- eine DNA-Sequenz (Spr), die für ein Signal-Peptid pre und/oder ein Peptid pro codiert,
- eine DNA-Sequenz (Scl), die für ein Peptid codiert, das eine Stelle zur proteolytischen Spaltung (Schneiden) durch das Produkt des gesamten Gens KEX2 oder eines Teils desselben aufweist, und
- eine DNA-Sequenz, die für das heterologe Protein codiert, das zusammen mit der Sequenz Spr den Vorläufer bildet,

dadurch gekennzeichnet, daß man in das Plasmid das gesamte Gen KEX2 oder einen Teil desselben inseriert, was zu einer Erhöhung der proteolytischen Aktivität der Endoprotease yscF führt.

2. Verfahren nach Anspruch 1, dadurch gekennzeichnet, daß es sich bei dem genannten Protein um Hirudin handelt.

3. Verfahren nach Anspruch 2, dadurch gekennzeichnet, daß es sich bei dem Hirudin um die Variante rHV2Lys47 handelt.

4. Verfahren nach einem der Ansprüche 1 bis 3, dadurch gekennzeichnet, daß der Teil des Gens KEX2 die Sequenz aufweist, die in der Fig. 2 dargestellt ist.

5. Verfahren nach Anspruch 4, dadurch gekennzeichnet, daß der Teil des Gens KEX2 die funktionelle Sequenz des Gens KEX2 zwischen den Basen Nr. 1349 und 3793 in der Fig. 2 aufweist.

6. Verfahren nach Anspruch 5, dadurch gekennzeichnet, daß der Teil des Gens KEX2 die funktionelle Sequenz des Gens KEX2 zwischen den Basen Nr. 1349 und 3488 in der Fig. 2 aufweist.

7. Verfahren nach Anspruch 4, dadurch gekennzeichnet, daß der Teil des Gens KEX2 zwischen den Basen Nr. 538 und 4010 in der Fig. 2 angeordnet ist und daß daraus die Sequenz zwischen den Basen Nr. 3488 und 3790 in der Fig. 2 deletiert ist.

8. Verfahren nach einem der Ansprüche 4 bis 7, dadurch gekennzeichnet, daß der Teil des Gens KEX2 an dem 3'-Ende ein Terminator-Fragment aufweist, das der Sequenz zwischen den Basen Nr. 3790 und 4010 in der Fig. 2 entspricht.

9. Verfahren nach Anspruch 8, dadurch gekennzeichnet, daß die Sequenz zwischen den Basen Nr. 3790 und 4010 in der Fig. 2 eine Stabilisatorrolle spielt.

10. Verfahren nach einem der vorhergehenden Ansprüche, dadurch gekennzeichnet, daß die Sequenz Scl eine Sequenz ist, die für ein Peptid Lys-Arg oder Arg-Arg codiert.

11. Verfahren nach einem der Ansprüche 1 bis 10, dadurch gekennzeichnet, daß die Sequenz Str ein starker Promotor in der Hefe ist.

12. Verfahren nach Anspruch 11, dadurch gekennzeichnet, daß es sich dabei um den Promotor des α-Pheromons von Hefe handelt.

13. Verfahren nach einem der Ansprüche 2 bis 11, dadurch gekennzeichnet, daß die Sequenz Spr die Sequenz pre-pro des Gens MFalpha1 von Hefe ist.

14. Verfahren nach Anspruch 13, dadurch gekennzeichnet, daß auf die Sequenz Spr eine Sequenz folgt, die für ein Peptid Lys-Arg oder Arg-Arg codiert.

15. Verfahren zur Herstellung eines Hefestammes, dadurch gekennzeichnet, daß er durch ein Plasmid, wie es in einem der Ansprüche 1 bis 14 erhalten wird, transformiert wird.

16. Stamm nach Anspruch 15, dadurch gekennzeichnet, daß es sich dabei um einen Stamm von Saccharomyces cerevisiae handelt.

17. Verfahren zur Herstellung von reifem Hirudin durch Hefen, dadurch gekennzeichnet, daß man in einem geeigneten Medium einen Hefestamm nach einem der Ansprüche 15 bis 16 kultiviert (züchtet) und das erhaltene reife Hirudin daraus gewinnt (abtrennt).

18. Verfahren nach Anspruch 17, dadurch gekennzeichnet, daß es sich bei dem Hirudin um die Variante rHV2Lys47 handelt.

**Claims**

**Claims for the following Contracting States : DE, GB, FR, IT, NL, SE, LI, CH, BE, AT, LU, DK**

1. Multicopy plasmid which is replicable in a yeast, comprising at least the following block for expression of a heterologous protein:
   - a DNA sequence (Str) containing the signals providing for transcription by the yeast of the sequence coding for a precursor of the maturable protein,
   - a DNA sequence (Spr) coding for a pre signal peptide and/or a pro peptide,
   - a DNA sequence (Scl) coding for a peptide comprising a site for proteolytic cleavage by the product of all or part of the KEX2 gene,
   - a DNA sequence coding for the heterologous protein, which, with the Spr sequence, forms the precursor,
   characterized in that the plasmid contains all or part of the KEX2 gene leading to an increase in the proteolytic activity of the endoprotease yscF.

2. Plasmid according to Claim 1, characterized in that the said protein is hirudin.

3. Plasmid according to Claim 2, characterized in that the hirudin is the variant rHV2Lys47.

4. Plasmid according to one of Claims 1 to 3, characterized in that it contains the portion of the KEX2 gene whose sequence is shown in Figure 2.

5. Plasmid according to Claim 4, characterized in that it contains the functional sequence of the KEX2 gene, between the bases numbered 1349 and 3793 in Figure 2.

6. Plasmid according to Claim 5, characterized in that it contains the functional sequence of the KEX2 gene between the bases numbered 1349 and 3488 in Figure 2.

7. Plasmid according to Claim 4, characterized in that it contains the portion of the KEX2 gene between the bases numbered 538 and 4010 in Figure 2, and in that the sequence between the bases numbered 3488 and 3790 in Figure 2 has been deleted therefrom.

8. Plasmid according to one of Claims 4 to 7, characterized in that the segment of the KEX2 gene contains, at the 3' end, a terminator fragment corresponding to the sequence between the bases numbered 3790 and 4010 in Figure 2.

9. Plasmid according to Claim 8, characterized in that the sequence between the bases numbered 3790 to 4010 in Figure 2 plays the part of a stabilizer.

10. Plasmid according to one of the preceding claims, characterized in that the Scl sequence is a sequence coding for a Lys-Arg or Arg-Arg peptide.

11. Plasmid according to one of Claims 1 to 10, characterized in that the Str sequence is a strong promoter in yeast.

12. Plasmid according to Claim 11, characterized in that the promoter in question is the promoter of the alpha pheromone of yeast.

13. Plasmid according to one of Claims 2 to 11, characterized in that the Spr sequence is the prepro sequence of the MFalphal gene of yeast.

14. Plasmid according to Claim 13, characterized in that the Spr sequence is followed by a sequence coding for a Lys-Arg or Arg-Arg peptide.

15. Yeast strain, characterized in that it contains a plasmid according to one of Claims 1 to 14.

**16.** Strain according to Claim 15, characterized in that it is a strain of <u>Saccharomyces cerevisiae.</u>

**17.** Process for preparing mature hirudin by means of yeasts, characterized in that a yeast strain according to one of Claims 15 to 16 is cultured in a suitable medium and the mature hirudin obtained is recovered.

**18.** Process according to Claim 17, characterized in that the hirudin is the variant rHV2Lys47.

**Claims for the following Contracting States : ES, GR**

**1.** Process for preparing a multicopy plasmid which is replicable in a yeast, comprising at least the following block for expression of a heterologous protein:
- a DNA sequence (Str) containing the signals providing for transcription by the yeast of the sequence coding for a precursor of the maturable protein,
- a DNA sequence (Spr) coding for a pre signal peptide and/or a pro peptide,
- a DNA sequence (Scl) coding for a peptide comprising a site for proteolytic cleavage by the product of all or part of the KEX2 gene,
- a DNA sequence coding for the heterologous protein, which, with the Spr sequence, forms the precursor,
characterized in that all or part of the KEX2 gene is inserted into the plasmid, leading to an increase in the proteolytic activity of the endoprotease yscF.

**2.** Process according to Claim 1, characterized in that the said protein is hirudin.

**3.** Process according to Claim 2, characterized in that the hirudin is the variant rHV2Lys47.

**4.** Process according to one of Claims 1 to 3, characterized in that the portion of the KEX2 gene has the sequence which is shown in Figure 2.

**5.** Process according to Claim 4, characterized in that the portion of the KEX2 gene contains the functional sequence of the KEX2 gene, between the bases numbered 1349 and 3793 in Figure 2.

**6.** Process according to Claim 5, characterized in that the portion of the KEX2 gene contains the functional sequence of the KEX2 gene between the bases numbered 1349 and 3488 in Figure 2.

**7.** Process according to Claim 4, characterized in that the portion of the KEX2 gene is between the bases numbered 538 and 4010 in Figure 2, and in that the sequence between the bases numbered 3488 and 3790 in Figure 2 has been deleted therefrom.

**8.** Process according to one of Claims 4 to 7, characterized in that the portion of the KEX2 gene contains, at the 3' end, a terminator fragment corresponding to the sequence between the bases numbered 3790 and 4010 in Figure 2.

**9.** Process according to Claim 8, characterized in that the sequence between the bases numbered 3790 and 4010 in Figure 2 plays the part of a stabilizer.

**10.** Process according to one of the preceding claims, characterized in that the Scl sequence is a sequence coding for a Lys-Arg or Arg-Arg peptide.

**11.** Process according to one of Claims 1 to 10, characterized in that the Str sequence is a strong promoter in yeast.

**12.** Process according to Claim 11, characterized in that the promoter in question is the promoter of the alpha pheromone of yeast.

**13.** Process according to one of Claims 2 to 11, characterized in that the Spr sequence is the prepro sequence of the MFalpha1 gene of yeast.

**14.** Process according to Claim 13, characterized in that the Spr sequence is followed by a sequence coding for a Lys-Arg or Arg-Arg peptide.

15. Process for preparing a yeast strain, characterized in that it is transformed with a plasmid obtained according to one of Claims 1 to 14.

16. Strain according to Claim 15, characterized in that it is a strain of <u>Saccharomyces cerevisiae</u>.

17. Process for preparing mature hirudin by means of yeasts, characterized in that a yeast strain obtained according to one of Claims 15 to 16 is cultured in a suitable medium and the mature hirudin obtained is recovered.

18. Process according to Claim 17, characterized in that the hirudin is the variant rHV2Lys47.

# FIG.1

| | 1 | 2 | 3 | 4 | 5 | 6 | 7 | 8 | 9 | 10 | 11 | 12 | 13 | 14 | 15 |
|---|---|---|---|---|---|---|---|---|---|---|---|---|---|---|---|
| 1 HV1 | VAL | VAL | TYR | THR | ASP | CYS | THR | GLU | SER | GLY | GLN | ASN | LEU | CYS | LEU |
| 2 HV2 | ILE | THR | TYR | THR | ASP | CYS | THR | GLU | SER | GLY | GLN | ASN | LEU | CYS | LEU |
| 3 HV3 | ILE | THR | TYR | THR | ASP | CYS | THR | GLU | SER | GLY | GLN | ASN | LEU | CYS | LEU |

| 16 | 17 | 18 | 19 | 20 | 21 | 22 | 23 | 24 | 25 | 26 | 27 | 28 | 29 | 30 |
|---|---|---|---|---|---|---|---|---|---|---|---|---|---|---|
| CYS | GLU | GLY | SER | ASN | VAL | CYS | GLY | GLN | GLY | ASN | LYS | CYS | ILE | LEU |
| CYS | GLU | GLY | SER | ASN | VAL | CYS | GLY | LYS | GLY | ASN | LYS | CYS | ILE | LEU |
| CYS | GLU | GLY | SER | ASN | VAL | CYS | GLY | LYS | GLY | ASN | LYS | CYS | ILE | LEU |

| 31 | 32 | 33 | 34 | 35 | 36 | 37 | 38 | 39 | 40 | 41 | 42 | 43 | 44 | 45 | 46 | 47 |
|---|---|---|---|---|---|---|---|---|---|---|---|---|---|---|---|---|
| GLY | SER | ASP | GLY | GLU | LYS | ASN | GLN | CYS | VAL | THR | GLY | GLU | GLY | THR | PRO | LYS |
| GLY | SER | ASN | GLY | LYS | GLY | ASN | GLN | CYS | VAL | THR | GLY | GLU | GLY | THR | PRO | ASN |
| GLY | SER | GLN | GLY | LYS | ASP | ASN | GLN | CYS | VAL | THR | GLY | GLU | GLY | THR | PRO | LYS |

| 48 | 49 | 50 | 51 | 52 | 53 | 54 | 55 | 56 | 57 | 58 | 59 | 60 | 61 | 62 | 63 | 64 | 65 |
|---|---|---|---|---|---|---|---|---|---|---|---|---|---|---|---|---|---|
| PRO | GLN | SER | HIS | ASN | ASP | GLY | ASP | PHE | GLU | GLU | ILE | PRO | GLU | GLU | TYR | LEU | GLN |
| PRO | GLU | SER | HIS | ASN | ASN | GLY | ASP | PHE | GLU | GLU | ILE | PRO | GLU | GLU | TYR | LEU | GLN |
| PRO | GLN | SER | HIS | ASN | GLN | GLY | ASP | PHE | GLU | PRO | ILE | PRO | GLU | ASP | TYR | ASP | GLU |

64 65 66

↓
ALA
63

1. D'après DODT et al. FEBS LETTERS 1984 165, 180-183.
2. D'après HARVEY et al. Proc. Natl. Acad. USA 1986 83, 1084-1088
3. D'après DODT et al. Biol. Chem. Hoppe-Seyler 1986 367, 803-811.

# FIG_2

```
  1           10          20          30          40          50
  .SmaI        .           .           .           .           .
  CCCGGGAATC  TCGGTCGTAA  TGATTTTTAT  AATGACGAAA  AAAAAAAAAT

  TGGAAAGAAA  AAGCTTTAAT  GCGGTAGTTT  ATCACAGTTA  AATTGCTAAC

100- GCAGTCAGGC  ACCGTGTATG  AAATCTAACA  ATGCGCTCAT  CGTCATCCTC

  GGCACCGTCA  CCCTGGATGC  TGTAGGCATA  GGCTTGGTTA  TGCCGGTACT

200- GCCGGGCCTC  TTGCGGGATA  TCGTCCATTC  CGACAGCATC  GCCAGTCACT

  ATGGCGTGCT  GCTAGCGCTA  TATGCGTTGA  TGCAATTTCT  ATGCGCACCC

300- GTTCTCGGAG  CACTGTCCGA  CCGCTTTGGC  CGCCGCCCAG  TCCTGCTCGC
                                                 Sau3A
  TTCGCTACTT  GGAGCCACTA  TCGACTACGC  GATCATGGCG  ACCACACCCG

400- TCCTGTGGAT  CTTGGATATA  CTTCAAAGCC  GACTATGGTA  TTTTTATCAT

  TATCACTGGC  ATGAAAACGT  ATTACCAGCA  TTACGTGATT  ATGCAGATAC
                                                 XhoI
500- GTTTTATCTG  TATCAGGATC  AAATCGTTAC  CTTCATCCTC  GAGCACAACA

  ACTGCTACTG  CCTCCGCCAA  CGATAACGGA  GCAACTTCAA  ACATCAATGG

600- ACAAGATCAC  TGGCTGCCAC  GTCGATGAAA  CGTCTAAATT  ACCACTTCGC

  TACCGCGTTG  AAAATTCCTG  GGTAAAGAC  TCCGGTAAAG  ACGGATTATA

700- CGTGATGACT  CAAAAGTACT  TCGAGGAGTA  CTGCTTTCAA  ATTGTGGTCG

  ATATCAATGA  ATTGCCAAAA  GAGCTGGCTT  CAAAATTCAC  CTCAGGTAAG

800- GAAGAGCCGA  TTGTCTTGCC  CATCTGGGAC  CCAATGGGTG  CTTTGGCCAA

  ATAAATAGTT  TCAGCAGCTC  TGATGTAGAT  ACACGTATCT  CGACATGTTT

900- TATTTTTACT  ATACATACAT  AAAAGAAATA  AAAAATGATA  ACGTGTATAT

  TATTATTCAT  ATAATCAATG  AGGGTCATTT  TCTGAAACGC  AAAAAACGGT

1000- AAATGGAAAA  AAAATAAAGA  TAGAAAAAGA  AAACAAACAA  AGGAAAGGTT

  AGCATATTAA  ATAACTGAGC  TGATACTTCA  ACAGCATCGC  TGAAGAGAAC

1100- AGTATTGAAA  CCGAAACATT  TTCTAAAGGC  AAACAAGGTA  CTCCATATTT

  GCTGGACGTG  TTCTTTCTCT  CGTTTCATAT  GCATAATTCT  GTCATAAGCC

1200- TGTTCTTTTT  CCTGGCTTAA  ACATCCCGTT  TTGTAAAAGA  GAAATCTATT
```

16

```
        1         10        20        30        40        50
        .         .         .         .         .         .
        CCACATATTT CATTCATTCG GCTACCATAC TAAGGATAAA CTAATCCCGT

 1300-  TGTTTTTTGG CCTCGTCACA TAATTATAAA CTACTAACCC ATTATCAGAT
                                                            start
        GAAAGTGAGG AAATATATTA CTTTATGCTT TTGGTGGGCC TTTTCAACAT

 1400-  CCGCTCTTGT ATCATCACAA CAAATTCCAT TGAAGGACCA TACGTCACGA

        CAGTATTTTG CTGTAGAAAG CAATGAAACA TTATCCCGCT TGGAGGAAAT

 1500-  GCATCCAAAT TGGAAATATG AACATGATGT TCGAGGGCTA CCAAACCATT

        ATGTTTTTTC AAAAGAGTTG CTAAAATTGG GCAAAAGATC ATCATTAGAA

 1600-  GAGTTACAGG GGGATAACAA CGACCACATA TTATCTGTCC ATGATTTATT

        CCCGCGTAAC GACCTATTTA AGAGACTACC GGTGCCTGCT CCACCAATGG

 1700-  ACTCAAGCTT GTTACCGGTA AAAGAAGCTG AGGATAAACT CAGCATAAAT

        GATCCGCTTT TTGAGAGGCA GTGGCACTTG GTCAATCCAA GTTTTCCTGG

 1800-  CAGTGATATA AATGTTCTTG ATCTGTGGTA CAATAATATT ACAGGCGCAG

        GGGTCGTGGC TGCCATTGTT GATGATGGCC TTGACTACGA AAATGAAGAC

 1900-  TTGAAGGATA ATTTTTGCGC TGAAGGTTCT TGGGATTTCA ACGACAATAC

        CAATTTACCT AAACCAAGAT TATCTGATGA CTACCATGGT ACGAGATGTG

 2000-  CAGGTGAAAT AGCTGCCAAA AAAGGTAACA ATTTTTGCGG TGTCGGGGTA

        GGTTACAACG CTAAAATCTC AGGCATAAGA ATCTTATCCG GTGATATCAC

 2100-  TACGGAAGAT GAAGCTGCGT CCTTGATTTA TGGTCTAGAC GTAAACGATA

        TATATTCATG CTCATGGGGT CCCGCTGATG ACGGAAGACA TTTACAAGGC

 2200-  CCTAGTGACC TGGTGAAAAA GGCTTTAGTA AAAGGTGTTA CTGAGGGAAG

        AGATTCCAAA GGAGCGATTT ACGTTTTTGC CAGTGGAAAT GGTGGAACTC

 2300-  GTGGTGATAA TTGCAATTAC GACGGCTATA CTAATTCCAT ATATTCTATT

        ACTATTGGGG CTATTGATCA CAAAGATCTA CATCCTCCTT ATTCCGAAGG

 2400-  TTGTTCCGCC GTCATGGCAG TCACGTATTC TTCAGGTTCA GGCGAATATA

        TTCATTCGAG TGATATCAAC GGCAGATGCA GTAATAGCCA CGGTGGAACG

 2500-  TCTGCGGCTG CTCCATTAGC TGCCGGTGTT TACACTTTGT TACTAGAAGC
```

17

```
       1         10        20        30        40        50
       .         .         .         .         .         .
       CAACCCAAAC CTAACTTGGA GAGACGTACA GTATTTATCA ATCTTGTCTG

2600-  CGGTAGGGTT AGAAAAGAAC GCTGACGGAG ATTGGAGAGA TAGCGCCATG

       GGGAAGAAAT ACTCTCATCG CTATGGCTTT GGTAAAATCG ATGCCCATAA

2700-  GTTAATTGAA ATGTCCAAGA CCTGGGAGAA TGTTAACGCA CAAACCTGGT

       TTTACCTGCC AACATTGTAT GTTTCCCAGT CCACAAACTC CACGGAAGAG

2800-  ACATTAGAAT CCGTCATAAC CATATCAGAA AAAAGTCTTC AAGATGCTAA

       CTTCAAGAGA ATTGAGCACG TCACGGTAAC TGTAGATATT GATACAGAAA

2900-  TTAGGGGAAC TACGACTGTC GATTTAATAT CACCAGCGGG GATAATTTCA

       AACCTTGGCG TTGTAAGACC AAGAGATGTT TCATCAGAGG GATTCAAAGA

3000-  CTGGACATTC ATGTCTGTAG CACATTGGGG TGAGAACGGC GTAGGTGATT

       GGAAAATCAA GGTTAAGACA ACAGAAAATG GACACAGGAT TGACTTCCAC

3100-  AGTTGGAGGC TGAAGCTCTT TGGGGAATCC ATTGATTCAT CTAAAACAGA

       AACTTTCGTC TTTGGAAACG ATAAAGAGGA GGTTGAACCA GCTGCTACAG

3200-  AAAGTACCGT ATCACAATAT TCTGCCAGTT CAACTTCTAT TTCCATCAGC

       GCTACTTCTA CATCTTCTAT CTCAATTGGT GTGGAAACGT CGGCCATTCC

3300-  CCAAACGACT ACTGCGAGTA CCGATCCTGA TTCTGATCCA AACACTCCTA

       AAAAACTTTC CTCTCCTAGG CAAGCCATGC ATTATTTTTT AACAATATTT

3400-  TTGATTGGCG CCACATTTTT GGTGTTATAC TTCATGTTTT TTATGAAATC
                                                         EcoRI
       AAGGAGAAGG ATCAGAAGGT CAAGAGCGGA AACGTATGAA TTCGATATCA
                                                       * délétion
3500-  TTGATACAGA CTCTGAGTAC GATTCTACTT TGGACAATGG AACTTCCGGA

       ATTACTGAGC CCGAAGAGGT TGAGGACTTC GATTTTGATT TGTCCGATGA

3600-  AGACCATCTT GCAAGTTTGT CTTCATCAGA AAACGGTGAT GCTGAACATA

       CAATTGATAG TGTACTAACA AACGAAAATC CATTTAGTGA CCCTATAAAG

3700-  CAAAAGTTCC CAAATGACGC CAACGCAGAA TCTGCTTCCA ATAAATTACA

       AGAATTACAG CCTGATGTTC CTCCATCTTC CGGACGATCG TGATTCGATA
            HindIII                        délétion  * stop
3800-  TGTACAGAAA GCTTCAAATT ACAAAATAGC ATTTTTTTCT TATAGATTAT
```

```
        1        10        20        30        40        50
        .         .         .         .         .         .
        AATACTCTCT CATACGTATA CGTATATGTG TATATGATAT ATAAACAAAC

3900-   ATTAATATCC TATTCCTTCC GTTTGAAATC CCTATGATGT ACTTTGCATT

        GTTTGCACCC GCGAATAAAA TGAAAACTCC GAACCGATAT ATCAAGCACA
                   BamHI
4000-   TAAAAGGGGA GGGTCCAATT AATGCATATT TAAGACCACA GCTGAATAAC

        TTTAAAACGG CAGACAAAC AAAAAATAGG TCGAATAAAC CTTACCTGCC

4100-   TAGAAGGAAT GACAGCAGCT AATAAGAATA TTGTCTTCGG ATTTTCCAGA

        TCCATTAGCG CAATTCTACT AATATGCTTT TTCTTTGAAA AAGTCTGCGG

4200-   TGATATGGAG CATGATATGG GCATGGATGA TACTTCGGGA TACACGAGGC

        CAGAAATTGT GCAGGCTGGG TCGAAATCTT TCCACTGGCT CTGCACTTTG

4300-   GGATTCTTGT TGCTTTTACC ATCCGTGGTG ACGTGCCTTT CGTTCGCTGG

        CAGGATATAT TCAGCTACCC TCTTACAATG CACTTGTGCC GTTTACGCTT

4400-   TCTTAGAAGC TGCCGTATTA AGATTCAAG ACAATGATGG GGTAGAAAAT

        AGAACTTCAA GGGGAACCGC ATGGTTTTTG GTGGGACTTA CTTGGATAAC

4500-   CTTATTCTTT GGTGGATTAG CTGGAGGAAC TGGTTTCTTA GTGAAAAGCA

        AGAGGTTGCA AACGTTCATA TCAAATGCAG GTGAGAAAAG GTTGTCATAT

4600-   ATCCATCGTG GTTTATCCTT TCTAACTGTT CTAACAGGTT GGGTTAAAGT

        CTGTTTGGCA CCTGTTGCGC TCTTTGGGTT TTGTAGAGAG GCACACACAG

4700-   GGCAATGCAT CGCTCATGGT ATCATGGGAT CC
```

FIG_3

**HindIII**

**HindIII**

| | | |
|---|---|---|
| rHV2 Lys47 | Prepro | Promoteur |
| | MFalpha1 | |
| | | URA3-d |

M13TG3839

**SalI**

**SphI**

| | | | |
|---|---|---|---|
| Term. | rHV2 Lys47 | Prepro | Promoteur |
| PGK | | MFalpha1 | |

M13TG3841

FIG.4

FIG.5

EP 0 396 436 B1